Europäisches Patentamt

European Patent Office

Office européen des brevets

(19)

(11) Publication number: 0 063 328
A2

## (12) EUROPEAN PATENT APPLICATION

(21) Application number: 82103048.3

(22) Date of filing: 08.04.82

(51) Int. Cl.³: A 61 K 37/24

(30) Priority: 10.04.81 JP 53221/81

(43) Date of publication of application:
27.10.82 Bulletin 82/43

(84) Designated Contracting States:
BE CH DE FR GB IT LI NL SE

(71) Applicant: Eisai Co., Ltd.
6-10, Koishikawa 4-chome Bunkyo-ku
Tokyo 112(JP)

(72) Inventor: Fumio, Kakimoto
5-133-3, Mitsuike-cho Unuma
Kagamigahara-shi Gifu Prefecture(JP)

(72) Inventor: Naoki, Asakawa
105-2, Nakaoida-cho
Kagamigahara-shi Gifu Prefecture(JP)

(72) Inventor: Yasuo, Ishibashi
880-86, Nagaraobusa
Gifu Prefecture(JP)

(72) Inventor: Aishin, Shinoda
1-292, Aza-fusoudai Oaza-takao
Fusou-cho, Niwa-gun Aichi Prefecture(JP)

(72) Inventor: Yasuo, Miyake
1-17, Aza-todomegi Hashizume
Inuyama-shi Aichi Prefecture(JP)

(74) Representative: Hansen, Bernd, Dr.rer.nat. et al,
Hoffmann . Eitle & Partner Patentanwälte
Arabellastrasse 4
D-8000 München 81(DE)

(54) Compositions containing secretin and method for preventing the adsorption of secretin.

(57) A secretin containing composition which contains secretin and a basic amino acid having an isoelectric point of 7 or higher, such as arginine, lysine, ornithine, histidine etc., either as a mixture in which both are co-present or as a combination in which both are present separately, and a method for preventing the adsorption of secretin which comprises adding a basic amino acid having an isoelectric point of 7 or higher to a secretin containing aqueous solution.

EP 0 063 328 A2

36 755 m/fg

COMPOSITIONS CONTAINING SECRETIN AND METHOD FOR PREVENTING

THE ADSORPTION OF SECRETIN

The present invention relates to compositions containing secretin and method for preventing the adsorption of secretin

Secretin is a kind of gastrointestinal hormone , that is, it is a pancreatic juice secreting hormone and is a useful substance in the treatment or diagnosis of various diseases relating to the pancreas and the gall bladder. Since this substance is one of hormones, its single dosage is a very small amount, e.g. several micrograms, and therefore this dosage must be strictly observed. In other words, there is a special circumstance that said substance must be accurately administered in a direct way.

However, on the other hand, it is well known that in general, proteins and peptides present in aqueous solution are instantly adsorbed to a vitreous substance, resulting in that the level is considerably lower than the amount originally pharmaceutically incorporated. For example, when insulin is injected into an infusion container, a considerable amount of insulin is instantly adsorbed to the surface of the glass, and furthermore this adsorption has the tendency to take place more easily where the concentration of insulin is low [YAKUZAIGAKU, Vol.39, 107-111 (1979)]. More specifically, peptide hormones, such as insulin,

- 2 -

are in a condition to be easily adsorbed to a glass container since the dosage is intrinsically restricted to a very small amount and furthermore the percent loss due to the adsorption is great as might be expected since the original content is small, resulting in that the dosage is inevitably greatly decreased. The reduction in amount due to the adsorption to a glass container is the same phenomenon as observed with secretin dealt with in the present invention (Chem. Pharm. Bull., 27(12) 3160 - 3163 (1979)). In other words, secretin is a peptic hormone, the dosage of which is also very small as in the case of insulin, and therefore, when it is present in aqueous solution, it is readily adsorbed to a glass container, and moreover the percent loss due to the adsorption is considerably high. For example, even though the necessary amount has been accurately charged into a container by carefully avoiding the adsorption to the container and others, when this is then transferred to a syringe or infused into an infusion container for mixed infusion with the infusion on actual administration, a considerable amount thereof is adsorbed to the glass wall of the syringe or the glass wall of the infusion container, thus exerting a great aggravating influence on the results.

From such an aspect, technical approaches for preventing the adsorption of secretin to a container have been eagerly sought. Nevertheless, no solution has been offered yet in the prior art.

As for secretin aqueous solutions, there have been patent applications, such as Japanese Patent Publication No. 29005/1975

- 3 -

and Japanese Patent Application Laid-open No. 98315/1979, and they disclose inventions aiming to present stable secretin aqueous solutions. However, none of them solves the afore-mentioned subject of the present invention.

More specifically, Japanese Patent Publication No. 29005/1975 discloses a process for producing a stable secretin-containing lyophilized powder, which process is characterized by using alanine as a carrier. Although said alanine is effective for carrying out lyophilization and also effective for maintaining the chemical stability of secretin, it does not lend itself to the prevention of the adsorption of secretin, which is the subject of the present invention.

On the other hand, Japanese Patent Application Laid-open No. 98315/1979 discloses a preparation for presenting a stable secretin aqueous solution, which is characterized in that the pH of the aqueous solution is adjusted to pH 3, as well as a process for preparation thereof, and said pH adjustment has nothing to do with the solution of the subject of the present invention.

Under such circumstances, we have been intensively studying technical approaches for preventing the adsorption of secretin in aqueous solution to a container and, as a result, have discovered that the expected purpose can be achieved by adding a basic amino acid to a secretin aqueous solution, thereby having accomplished the present invention.

Accordingly, a principal object of the present invention

- 4 -

is to provide a secretin containing composition which contains secretin and a basic amino acid having an isoelectric point of 7 or higher either as a mixture in which both are co-present or as a combination in which both are present separately.

Fig. 1 is described in the Section of the Results in Experimental Example 1;

Fig. 2 is described in the Section of the Results in Experimental Example 1, wherein the lines correspond to amino acid concentrations of 2 % (line -•-) and 1% (line -o-), respectively;

Fig. 3 is described in the Section of the Results in Experimental Example 1;

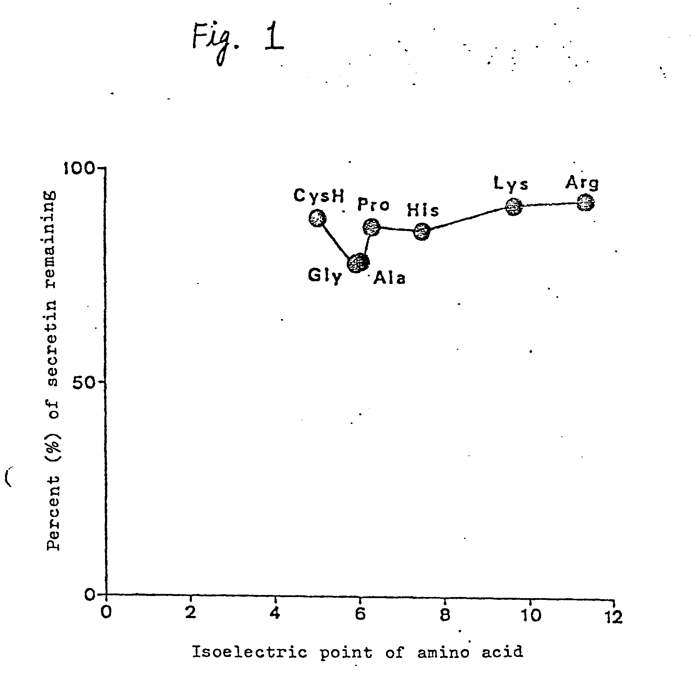

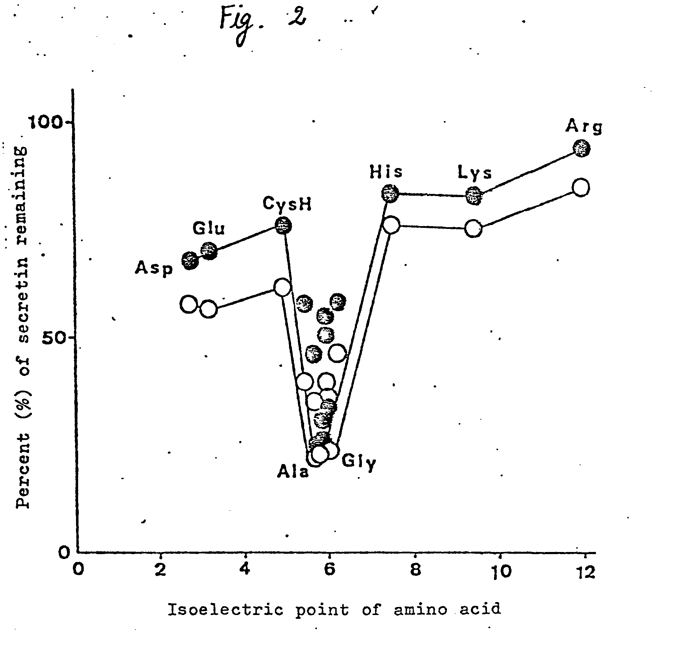

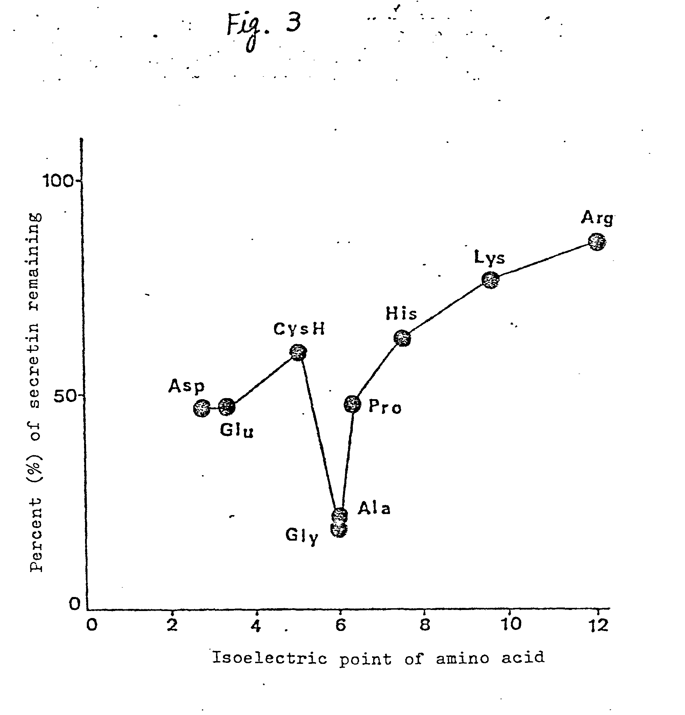

In Figs. 1 - 3, the symbols used have the meaning: - Asp for asparaginic acid, Glu for glutamic acid, CysH for cystein, Gly for glycine, Ala for alanine, Pro for Proline, His for histidine, Lys for lysine and Arg for arginine.

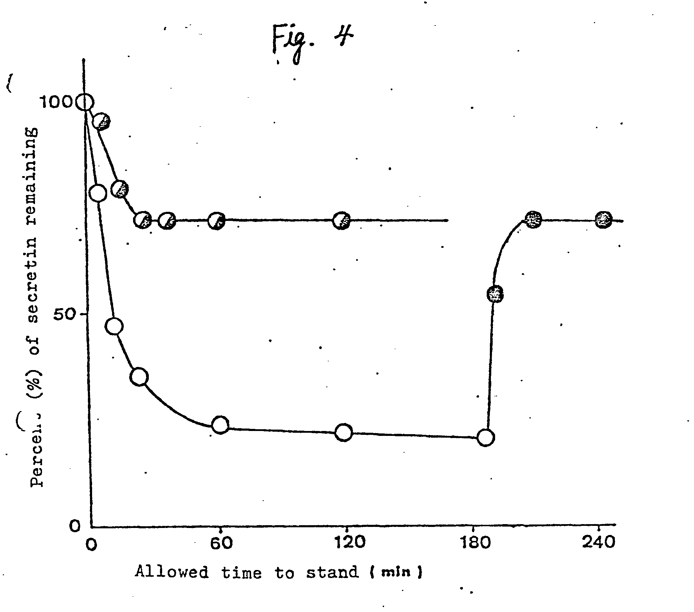

Fig. 4 is described in the Section of the Results in Experimental Example 2, wherein line -o- is for Experiment (A) before adding arginine, line -•- is for the same Experiment (A) but after adding arginine, and line -o- is for Experiment (B); and

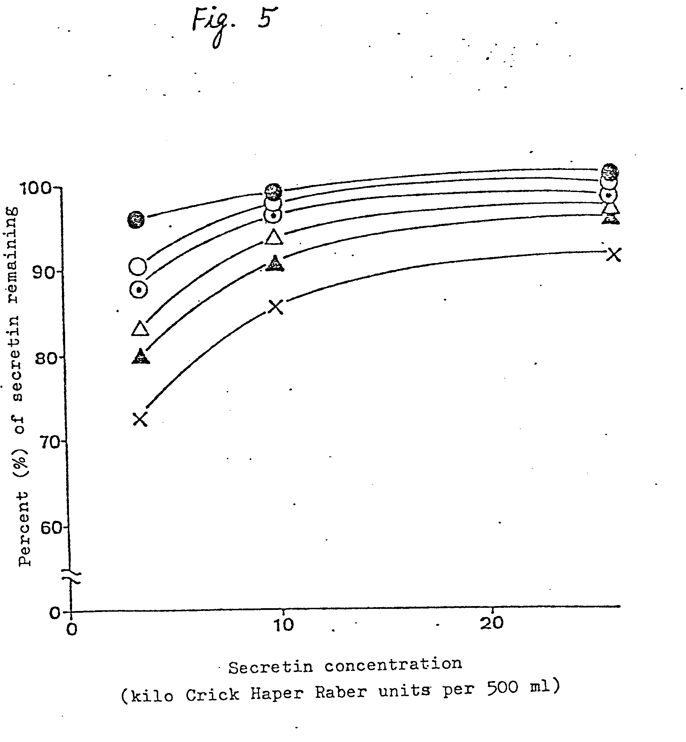

Fig. 5 is described in the Section of the Results in Experimental Example 3; wherein the lines correspond to concentrations of arginine added of 2% (line -•-), 1% (line -o-), 0.5% (line -⊙-), 0.2% (line -△-),

0.1% (line -▲-) and 0% (line -x-), respectively.

In the present invention, secretin may be one produced by any process, and in general that obtained by extraction, by means of several processes, from the small intestine mucous membrane of mammals, especially cattle, pigs etc., separating and purifying, is employed. While the dosage of secretin varies depending on the purpose of the diagnosis or treatment, a preparation containing 2000 - 5000 Crick Haper Raber units of secretin per ml is widely employed. However, the present invention is not restricted by the content of secretin.

The basic amino acid as used in the present invention is, as expressed according to the Experimental Examples described hereinbelow, that having an isoelectric point of 7 or higher. Therefore, specific examples may include histidine (isolectric point 7.47), lysine (do. 9.59), ornithine (do. 9.70) and arginine (do. 11.15).

On the other hand, the pH range of the secretin containing composition according to the present invention is 3 - 7. The pH of the secretin containing composition of the present invention is defined as follows: the secretin containing composition of the present invention is specifically presented in the form of an aqueous solution or a lyophilized powder, and therefore, if it is presented as the aqueous solution, the pH of said aqueous solution is defined as the pH of the seceretin containing composition of the present invention, whereas if the composition
is the lyophilized powder, the pH of an aqueous solution prepared by adding thereto a solution for dissolution is defined so.
In general, the pH of the secretin aqueous solution is usually
adjusted to a pH of 3 - 7, and therefore, also in the present
invention, the pH of the secretin containing composition defined
above is adjusted to the range of 3 - 7.

The afore-mentioned basic amino acid is, when at a pH in
the range of 3 - 7, in the state where said free amino acid and
its salt are co-present. Accordingly, the term "basic amino acids"
as used herein means, if more precisely defined, to include a
concept of not only the free basic amino acids but also their
salts. Therefore, the basic amino acid in the secretin containing composition of the present invention may be either one in
which said free amino acid and its salt are directly incorporated
at an appropriate ratio or that prepared by appropriately adding
other acidic substance to a free basic amino acid or appropriately adding other basic substance to a salt of said amino acid.
Accordingly, there is no particular restriction on choosing.

The amount of the basic amino acid incorporated is preferably such that when the secretin containing composition is
made into an aqueous solution, the concentration of said acid in
the aqueous solution is 0.1% or higher.

As will be clear from the Experimental Examples described
hereinbelow, the adsorption of secretin to a glass wall is
reversible. That is, secretin adsorbed to the glass wall
neither permanently remains adsorbed to the glass wall nor undergoes denaturation on the glass wall, but can desorb and again return into the aqueous solution, when a basic amino acid is added. Accordingly, it can be seen that the effect to prevent the adsorption by the basic amino acid may be brought about by the result of the competitive adsorption thereof with secretin to the glass wall. It may therefore be deduced that the minimum required amount of the basic amino acid is the amount determined not by the content of the secretin but by the surface area of the inner surface of the container. However, similarly to the case of insulin as described above, since the percent adsorption of secretin to the glass wall is high when the concentration of secretin is low, a great amount of the basic amino acid is necessary in order to maintain the predetermined concentration of secretin; on the contrary where the secretin concentration is high, the percent adsorption becomes low, whereby a small amount of the basic amino acid may be used.

The case in which the composition of the present invention is made into an aqueous solution by diluting in a container having a large surface area is a case where it is injected into a 500 ml infusion container. According to the Experimental Exam- ples described hereinbelow which had been conducted on the assumption of such a case, it was found that the miminum required amount of the basic amino acid is 0.1% and the more, the better. However, if the concentration of the basic amino acid is unnecessarily increased, the tension of the aqueous solution becomes too great to be practical, and thus the upper limit of the concentration is suitably 10% or so.

There are several pharmaceutical forms present as the compositions of the present invention. That is, the effect of the present invention is exerted by the so-called competitive adsorpotion as described above, and therefore it is not essential that secretin and the basic amino acid are originally present in the same composition. It is therefore possible to use such a composition form wherein both components are separately provided and may be mixed on use. Therefore, specific examples of the compositions include the following pharmaceutical forms:

i) A mixture in which secretin and a basic amino acid are co-present in the same aqueous solution..

ii) A combination in which secretin and a basic amino acid are in separate aqueous solutions and both may be mixed on use.

iii) A mixture in which secretin and a basic amino acid are co-present in the same lyophilized powder, and to which a separately prepared solution for dissolution may be added on use.

iv) A combination in which secretin and a basic amino acid are present as separate lyophilized powders and may be made into respective aqueous solutions and mixed on use.

v) A combination in which secretin is provided as a lyophilized powder and a basic amino acid is provided as an aqueous solution and both may be mixed on use.

The productions of the various aqueous solutions and lyophilized powders mentioned above may be easily carried out by conventional procedures, respectively. Further, it may also be freely practiced in the present invention to add an appropriate stabilizer, buffer etc. to the aqueous solutions, and to add a buffer to the lyophilized powders or an appropriate excipient or the like for lyophilizing.

The present invention also provides a method for preventing the adsorption of secretin to a glass wall in a secretin containing aqueous solution. More particularly, for the purpose of preventing the adsorption of secretin to a glass wall, the following methods also belong to the same technical idea as the aspect of the adsorption preventing composition of the present invention which has been described in detail hereinabove:

a method which comprises adding a separately prepared basic amino acid aqueous solution to a secretin containing infusion or a secretin containing ampule solution; or

a method which comprises adding secretin to an already prepared infusion or ampule solution containing the basic amino acid.

The effect of the present invention is described by the following Experimental Examples.

Experimental Example 1

To 50 ml of an aqueous solution buffered to pH 3.0 with a citrate phosphate buffer (M/100 citric acid – M/50 disodium phosphate) were added secretin and one of various amino acids having different isoelectric points at concentrations of 100 Crick Haper Raber units/ml and 2% respectively. To this were added 125 mg (corresponding to a surface area of about 15 $m^2$) of glass beads of a mesh size of 80/120 and a surface area of 118 $m^2/g$, and the mixture was shaken at 25°C for 2 hours. The amounts of secretin before and after the shaking with the glass beads were measured using . high speed liquid chromatography, to determine the percent remaining. In the high speed liquid chromatography, the measuring wavelength was 210 nm and the moving phase was $CH_3CN - H_2O - 70\%\ HClO_4$ $(40 : 60 : 0.5)$. These conditions for the high speed liquid chromatography were also used in the subsequent experimental examples.

Similar experiments were carried out except that in the above measurement, the pH buffered to 3.0 was replaced by pH 4.0 and 6.0 respectively, and the percents remaining in the cases of pH 4.0 and 6.0 were determined. Further, in the case of pH 4.0, another experiment was similarly carried out by changing the amino acid concentration of 2% to 1%, and the percent remaining was determined.

Results

The relationships between the isoelectric point of the amino acid added and the percent of secretin remaining are shown for the cases of pH 3.0, 4.0 and 6.0 in Fig. 1, Fig. 2 and Fig. 3, respectively.

For reference, in the cases of pH 3.0, 4.0 and 6.0, the percents remaining where no amino acid was added were also measured, to obtain 13%, 22% and 25%, respectively.

From these figures, it is found that amino acids having an isoelectric point of 7 or higher possess the adsorption preventing effect of the present invention. In general, since it is usual to adjust the pH of the secretin aqueous solution to pH 3 - 7, desirably pH 4 - 6, the basic amino acids having an isoelectric point of 7 or higher can exhibit the effect of the present invention in said pH range.

- 11 -

Experimental Example 2

(A)  To 50 ml of an aqueous solution buffered to pH 4.0 with a citrate phosphate buffer (M/100 citric acid - M/50 disodium phosphate) was added secretin to a concentration of 100 Crick Haper Raber units/ml.  To this were added 125 mg (corresponding to a surface area·of about 15 $m^2$) of glass beads of a mesh size of 80/120 and a surface area of 118 $m^2/g$, and the mixture was shaken at 25°C for 3 hours, during which the amount of secretin was periodically measured using the high speed liquid chromatography, to determine the percent remaining.  Thereafter, arginine was·added to this solution to a concentration of 0.5%, and the amount of secretin in the aqueous solution was again periodically measured.

(B)  To 50 ml of an aqueous solution buffered to pH 4.0 with a citrate phosphate buffer (M/100 citric acid - M/50 disodium phosphate) were added secretin and arginine to concentrations of 100 Crick Haper Raber units/ml and 0.5% respectively.  To this were added 125 mg (corresponding to a surface area of about 15 $m^2$) of glass beads of a mesh size of 80/120· and a surface area of 118 $m^2/g$, and the mixture was shaked at 25°C, and the amount of secretin was periodically measured using the high speed liquid chromatography, to determine the percent remaining.

Results

The results are shown in Fig. 4.  From Fig. 4, it can be seen that the adsorption of secretin to a glass wall is reversible.  That is, it is found that the once adsorbed secretin easily desorbs by the addition of arginine and reaches the same percent remaining

- 12 -

as that where arginine has been added at the start.

Experimental Example 3

To 500 ml of a 5% xylit infusion (contained in an infusion bottle) were added secretin and arginine at various concentrations, and each solution obtained was allowed to stand for 2 hours. The amounts of secretin before and after the standing were measured using the high speed liquid chromatography, to determine the percent remaining after the standing.

Results

The results are shown in Fig. 5. From Fig. 5, it can be seen that the minimum required concentration of arginine is 0.1%. That is, where the secretin concentration is low, e.g. up to 10 kilo Crick Haper Raber units per 500 ml, the arginine concentration required for ensuring a percent of secretin remaining of 90% or higher is 1% or higher. On the contrary, where the secretin concentration is high, e.g. 10 kilo Crick Haper Raber units per 500 ml or higher, the arginine concentration required for ensuring a percent secretin remaining of 90% or higher is 0.1% or higher. Since the secretin aqueous solution is employed at various concentrations, it can be said that the minimum required concentration of arginine necessary for manifesting the effect of the present invention is 0.1%, regardless of the concentration of secretin.

The present invention is more particularly described by the following examples.

- 13 -

EXAMPLE   1

An aqueous solution containing 2 g of L-alanine, 2 g of L-arginine hydrochloride and 5000 Crick Haber Raber units of secretin in 50 ml of a 0.03 M citrate phosphate buffer at pH 4.5 is aseptically prepared, dispensed 0.5 ml each into ampules, lyophilized and fusion sealed.  Separately, ampules each containing 1 ml of injectable distilled water are prepared to provide ampules of the solution for dissolution.

EXAMPLE   2

An aqueous solution containing 2 g of L-alanine, 0.2 g of L-cysteine and 5000 Crick Haper Raber units in 50 ml of a 0.03 M citrate phosphate buffer at pH 4.5 is aseptically prepared, dispensed 0.5 ml each into ampules, lyophilized and fusion sealed.  Separately, a 2% L-arginine hydrochloride aqueous solution is aseptically prepared, dispensed 2 ml each into ampules and fusion sealed to provide ampules of the solution for dissolution.

Claims

1. A secretin containing composition which contains secretin and a basic amino acid having an isoelectric point of 7 or higher either as a mixture in which both are co-present or as a combination in which both are present separately.

2. The secretin containing composition according to Claim 1 wherein the basic amino acid is chosen from arginine, lysine, ornithine, histidine and salts thereof.

3. The secretin containing composition according to Claim 1 or 2 wherein the amount of the basic amino acid incorporated is such that when said secretin containing composition is made into an aqueous solution, the concentration in said aqueous solution is 0.1 - 10%.

4. The secretin containing composition according to any of Claims 1 - 3 wherein said secretin containing composition is an aqueous solution.

5. The secretin containing composition according to any of Claims 1 - 3 wherein said secretin containing composition is a lyophilized powder.

6. The secretin containing composition according to any of Claims 1 - 3 wherein said secretin containing composition is a combination of a lyophilized powder and an aqueous solution.

7. A method for preventing the adsorption of secretin which is characterized by adding a basic amino acid having an isoelectric point of 7 or higher to a secretin containing aqueous solution.

8. The method for preventing the adsorption of secretin according to Claim 7 wherein the basic amino acid is chosen from arginine, lysine, ornithine, histidine and salts thereof.

9. The method for preventing the adsorption of secretin according to Claim 7 or 8 wherein the amount of the basic amino acid added is 0.1 - 10% of the secretin containing aqueous solution.

Fig. 1

12

Fig. 2

Percent (%) of secretin remaining

100

Arg

His    Lys

CysH

Glu

Asp

50

Ala    Gly

0

0    2    4    6    8    10    12

Isoelectric point of amino acid

Fig. 3

Fig. 4

Percent (%) of secretin remaining

100

50

0

0    60    120    180    240

Allowed time to stand ( min )

Fig. 5

Secretin concentration
(kilo Crick Haper Raber units per 500 ml)